# EUROPEAN PATENT APPLICATION

(11) **EP 4 553 471 A1**
(43) Date of publication of application: **14.05.2025**
(21) Application number: 23209174.4
(22) Date of filing: 10.11.2023
(51) Int. Cl.: G01M 3/40, G01N 27/20, A61J 1/06, A61M 5/00, A61M 5/178, A61M 5/32, A61M 5/50

(54) **SYSTEM AND METHOD FOR LEAK DETECTION IN A MEDICAL INJECTION DEVICE HAVING A RFID-TAGGED NEEDLE SHIELD**

(71) Applicant: Becton, Dickinson and Company, Franklin Lakes, NJ 07417-1880 (US)
(72) Inventor: RIVIER, Cédric, 38340 Voreppe (FR); THUILLIEZ, Julien, 38240 Meylan (FR); ROUDET, Theo, 38400 Saint-Martin-d'Heres (FR); GAGET, Emmanuel, 38000 Grenoble (FR)
(74) Representative: Germain Maureau

(57) **Abstract**

Provided herein is a system for detecting needle shield piercing in RFID-tagged medical injection devices. The system includes a conveyor system configured to convey the injection devices along a conveying path, a RFID coupling element positioned at a reading location along the conveying path, a RFID reader configured to perform a singulated reading of the RFID tag of each injection device upon passing the RFID coupling element, and a processor coupled to a memory and configured to record, for each RFID tag read by the RFID reader, a carrier power of a signal transmitted from the RFID coupling element to the RFID tag needed to generate a backscattered signal response from the RFID tag, compare the carrier power to a specified reference carrier power threshold and, if the recorded carrier power is greater than the reference carrier power threshold, identify the respective injection device as having a pierced needle shield.

## Description

### BACKGROUND OF THE INVENTION

### Field of the Invention

The present disclosure relates generally to RFID-tagged medical injection devices that include a needle shield and, more particularly, to a system and method for needle shield leak detection via reading of the RFID tag of the medical injection device.

### Description of Related Art

Syringes are used in a variety of environments for administering fluids, such as medications or other drugs, to a patient. Syringes may be provided as prefilled or pre-fillable syringes, which provide the convenience of rapidly delivering the liquid therein to a patient without the need to first aspirate the medication from another container and meter its volume. A syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

It is recognized that syringes must be handled with care before and after use due to the presence of the needle. To minimize the risk of accidental injury due to needle sticks, the syringe typically includes a needle shield that covers the sharpened tip of the needle. The needle shield is removed prior to use to expose the sharpened tip of the needle. Such a shield also serves to protect the sharpened tip of the needle and to preserve the sterility of the syringe content along with the syringe parts that come into contact with the patient skin. Needle shields are generally formed to include a rigid component and a soft component or "jupe." The soft component is typically formed of elastic rubber (e.g., isoprene rubber, butyl rubber, latex rubber, silicone rubber or the like) that provides a secure sealing connection with the syringe, at least along a sealing line, and the rigid component provides protection against accidental needle sticks and provides the user with an easily grippable surface for the user to remove the needle shield from the syringe.

During the manufacturing of syringes as described above, quality control checks/tests may be performed to ensure conforming of the syringe to set specifications. For example, quality control tests may be performed on the syringe to check if the needle shield has been pierced by the needle during placement thereof onto the syringe needle, with it recognized that such piercing of the needle shield may result in leakage that compromises the container closure integrity of the syringe. Existing systems and methods for detecting piercing (and associated leakage) of the needle shield typically employ a high voltage leak detection (HVLD) technique or a visual inspection technique, but it is recognized that each of these detection/inspection techniques has limitations or drawbacks associated therewith.

HVLD is a means to detect leaks in packaging through the application of high voltage. HVLD detection methods operate on the principles of electrical resistance and capacitance. To test a package such as a syringe needle shield using HVLD, the needle shield must be made of nonconductive materials (e.g., plastic and an elastomer) and the product inside (e.g., a medicament within the syringe barrel to which the shield is attached) must be conductive. A needle shield will be exposed to high voltage between at least two probes, where one probe applies high voltage to the needle shield and a second probe is a grounding source. If the needle shield has a leak, the electrical resistance of the needle shield walls is reduced, and voltage will conduct through the needle shield - with such voltage being detected/measured to identify a leak. However, it is recognized that in order to employ HVLD for testing the syringe needle shield, the medicament must have already been loaded/filled into the syringe at the time of testing, and this may limit the times/locations for testing during the manufacturing process.

The use of visual inspections for detecting leaks in a syringe needle shield is an inspection technique whereby the needle shield is inspected via a visual inspection system (e.g., image capture device and associated image processor). However, the use of a visual inspection system for identifying leak detection in a needle shield requires a clear line of sight to the needle shield and to the needle contained within the (transparent) needle shield, in order to ascertain positioning of the needle relative to the needle shield. It is recognized that some next-generation needle shields may incorporate an RFID tag therein that provides for individual identification and traceability of a syringe during/after manufacturing, but the RFID tag may at least partially encircle the needle shield, so as to block a line of sight between the visual inspection system and portions of the needle shield and/or to the needle within the needle shield. That is, an antenna of the RFID tag that is made from an electrically conductive material such as aluminum or copper may encircle parts of the needle shield, therefore blocking a line of sight to the needle and inhibiting the use of a visual inspection system for leak detection.

Accordingly, a need exists in the art for a system and method by which the needle shield may be inspected to detect leaks therein caused by piercing of the needle shield by the syringe needle. The system and method would desirably employ a leak detection technique other than HVLD or visual inspection leak detection, that enables leak detection at various points/times in the manufacturing cycle (e.g., prior to filling of syringe with a medicament) and/or with various types of syringe needle shields - and specifically for RFID-tagged needle shields. The system and method could beneficially be employed in addition to legacy HVLD or visual inspection systems to detect pierced needle shield defects when the pierced needle is completely hidden by the RFID tag antenna.

### SUMMARY OF THE INVENTION

Provided herein is a system for detecting needle shield piercing in each of a plurality of RFID-tagged medical injection devices having a needle shield with a RFID tag integrated therein. The system includes a conveyor system configured to convey the plurality of RFID-tagged medical injection devices along a conveying path, a RFID coupling element positioned at a reading location along the conveying path, a RFID reader operably connected with the RFID coupling element and configured to selectively perform a singulated reading of the RFID tag of each respective RFID-tagged medical injection device upon the RFID-tagged medical injection device passing the RFID coupling element at the reading location, and at least one processor coupled to a memory and configured to record, for each RFID tag read by the RFID reader, a carrier power of a signal transmitted from the RFID coupling element to the RFID tag needed to generate a backscattered signal response from the RFID tag, compare the carrier power provided to each respective RFID tag to a specified reference carrier power threshold and, if the carrier power provided to a respective RFID tag read by the RFID reader is greater than the reference carrier power threshold, identify the respective RFID-tagged medical injection device as having a pierced needle shield.

In accordance with some aspects of the disclosure, the reference carrier power threshold comprises a minimum carrier power needed to generate a backscattered signal from the RFID tag, for a RFID-tagged medical injection device having an intact needle shield.

In accordance with some aspects of the disclosure, the reference carrier power threshold comprises a carrier power curve of carrier power vs. frequency.

In accordance with some aspects of the disclosure, the reference carrier power threshold is -1.5dBm for a frequency of 865MHz, -5.5dBm for a frequency of 915MHz, and -1.5dBm for a frequency of 960MHz.

In accordance with some aspects of the disclosure, the at least one processor is further configured to determine the reference carrier power threshold from a plurality of reference readings acquired from RFID-tagged medical injection devices having an intact needle shield, the reference readings being carrier powers needed to generate a backscattered signal response from the RFID tag of the RFID-tagged medical injection devices.

In accordance with some aspects of the disclosure, the system further includes at least one trigger sensor configured to detect when an individual RFID-tagged medical injection device is at the reading location, wherein for each RFID-tagged medical injection device of the plurality of RFID-tagged medical injection devices on the conveyor system, the at least one sensor is configured to transmit a trigger signal to the RFID reader to cause the RFID reader to read the RFID tag of a respective RFID-tagged medical injection device in response to detecting that that RFID-tagged medical injection device is at the reading location, and wherein the RFID reader is configured to read the RFID tag on the respective RFID-tagged medical injection device in response to receiving the trigger signal from the at least one sensor.

In accordance with some aspects of the disclosure, in response to identifying a respective RFID-tagged medical injection device as having a pierced needle shield, the at least one processor is further configured to control the conveyor system to eject the RFID-tagged medical injection device from the conveyor system.

In accordance with some aspects of the disclosure, the plurality of RFID-tagged medical injection devices comprise a plurality of syringes.

In accordance with some aspects of the disclosure, the RFID coupling element comprises a near field coupling element placed from 1mm to 20mm from the RFID tag when a respective RFID-tagged medical injection device passes the reading location.

In accordance with some aspects of the disclosure, the conveyor system is configured to convey the plurality of RFID-tagged medical devices past the reading location at a rate of up to 1000 parts/min.

Also provided herein is a method for detecting needle shield piercing in each of a plurality of RFID-tagged medical injection devices having a needle shield with a RFID tag integrated therein. The method includes conveying each of a plurality of RFID-tagged medical devices, via a conveyor system, along a conveying path, each of the plurality of RFID-tagged medical devices comprising an RFID tag integrated with a needle shield thereof. The method also includes providing a RFID reading system at a position along the conveying path, the RFID reading system comprising a RFID coupling element positioned at a reading location along the conveying path and a RFID reader operably connected with the RFID coupling element. The method further includes performing a singulated reading of each of the plurality of RFID-tagged medical devices via the RFID reading system, as the respective RFID-tagged medical devices pass the reading location and, based on the reading of each of the plurality of RFID-tagged medical devices, identifying, via at least one processor, a potential needle shield piercing in each respective RFID-tagged medical injection device. In identifying a potential needle shield piercing, the method comprises recording, for each RFID tag, a carrier power of a signal transmitted from the RFID coupling element to the RFID tag needed to generate a backscattered signal response from the RFID tag, comparing the carrier power provided to each respective RFID tag to a specified reference carrier power threshold and, if the carrier power provided to a respective RFID tag read by the RFID reader is greater than the reference carrier power threshold, identifying the respective RFID-tagged medical injection device as having a pierced needle shield.

In accordance with some aspects of the disclosure, the reference carrier power threshold comprises a minimum carrier power needed to generate a backscattered signal from the RFID tag, for a RFID-tagged medical injection device having an intact needle shield.

In accordance with some aspects of the disclosure, the method further includes controlling the conveyor system to eject a respective RFID-tagged medical injection device from the conveyor system responsive to the RFID-tagged medical injection device being identified as having a pierced needle shield.

In accordance with some aspects of the disclosure, the method further includes detecting when an individual RFID-tagged medical injection device is at the reading location using at least one trigger sensor and, in response to detecting that that RFID-tagged medical injection device is at the reading location, transmitting a trigger signal from the at least one trigger sensor to the RFID reader to cause the RFID reader to read the RFID tag of a respective RFID-tagged medical injection device.

In accordance with some aspects of the disclosure, the method further includes positioning the RFID coupling element at the reading location so as to be spaced 1-20 mm from the RFID tag when a respective RFID-tagged medical injection device passes the reading location.

Also provided herein is a system for detecting needle shield piercing in each of a plurality of RFID-tagged medical injection devices having a needle shield with a RFID tag integrated therein. The system includes a conveyor system configured to convey the plurality of RFID-tagged medical injection devices along a conveying path, a RFID coupling element positioned at a reading location along the conveying path, a RFID reader operably connected with the RFID coupling element and configured to selectively perform a singulated reading of the RFID tag of each respective RFID-tagged medical injection device upon the RFID-tagged medical injection device passing the RFID coupling element at the reading location, and at least one processor coupled to a memory and configured to determine a tag sensitivity of the RFID tag in each of the RFID-tagged medical injection devices read by the RFID reader, compare the tag sensitivity of each RFID tag to a pre-determined normal tag sensitivity range and, if the tag sensitivity for a respective RFID tag is outside of the normal tag sensitivity range, identify the respective RFID-tagged medical injection device as having a pierced needle shield.

In accordance with some aspects of the disclosure, the at least one processor is further configured to determine the normal tag sensitivity range from a plurality of reference readings acquired from RFID-tagged medical injection devices having an intact needle shield.

In accordance with some aspects of the disclosure, in determining the tag sensitivity, the at least one processor is configured to record, for each RFID tag read by the RFID reader, a carrier power of a signal transmitted from the RFID coupling element to the RFID tag needed to generate a backscattered signal response from the RFID tag, and wherein in comparing the tag sensitivity of each RFID tag to the pre-determined normal tag sensitivity range, the at least one processor is configured to compare the carrier power to a specified reference carrier power threshold, with the respective RFID-tagged medical injection device being identified as having a pierced needle shield when the recorded carrier power for the respective RFID tag read by the RFID reader is greater than the reference carrier power threshold.

In accordance with some aspects of the disclosure, in response to identifying a respective RFID-tagged medical injection device as having a pierced needle shield, the at least one processor is further configured to control the conveyor system to eject the RFID-tagged medical injection device from the conveyor system.

In accordance with some aspects of the disclosure, the RFID coupling element comprises a near field coupling element placed from 1mm to 20mm from the RFID tag when a respective RFID-tagged medical injection device passes the reading location.

### BRIEF DESCRIPTION OF THE DRAWINGS

FIG. 1A is a perspective view of a RFID-tagged syringe, with which embodiments of the disclosure may be implemented;
FIG. 1B is an exploded view of the syringe of FIG. 1A;
FIG. 1C is a side view of a needle shield included in the syringe of FIG. 1A;
FIG. 2 illustrates an environment in which a method of reading and testing a plurality of RFID-tagged medical injection devices, such as the syringe of FIG. 1A, may be performed, according to a non-limiting embodiment described herein;
FIG. 3 is a diagram of non-limiting embodiments or aspects of components of one or more devices and/or one or more systems of FIG. 4; and
FIG. 4 is a flowchart of a process for detecting needle shield piercing in a RFID-tagged medical injection device, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

As used herein, the terms "communication" and "communicate" may refer to the reception, receipt, transmission, transfer, provision, and/or the like of information (e.g., data, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices that are configured to directly or indirectly communicate with or over one or more networks. A computing device may be a mobile or portable computing device, a desktop computer, a server, and/or the like that is specifically configured to provide one or more of the features and/or functions described herein. Furthermore, the term "computer" may refer to a specifically configured computing device that includes the necessary components to receive, process, and output data according to aspects described herein, and normally includes a display, a processor, a memory, an input device, and a network interface. A "computing system" may include one or more computing devices or computers. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.). Further, multiple computers, e.g., servers, or other computerized devices directly or indirectly communicating in the network environment may constitute a "system" or a "computing system".

As used herein, the term "communication network" may refer to one or more wired and/or wireless networks. For example, a communication network may include a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN)), a private network, an ad hoc network, an intranet, the Internet, a fiber optic-based network, a cloud computing network, and/or the like, and/or a combination of these or other types of networks.

It will be apparent that systems and/or methods, described herein, can be implemented in different forms of hardware, firmware, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

Some non-limiting embodiments or aspects are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, within a range specified by a threshold, etc.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise.

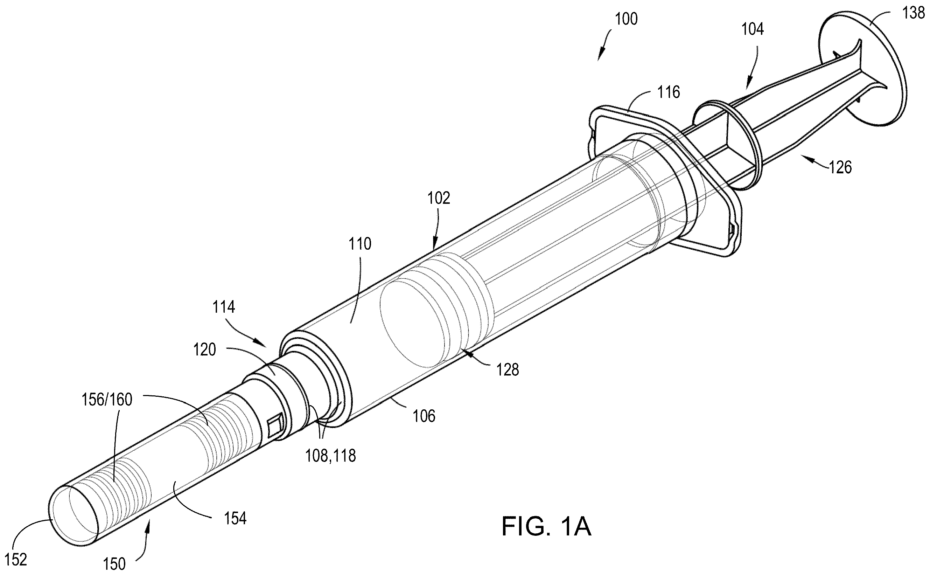

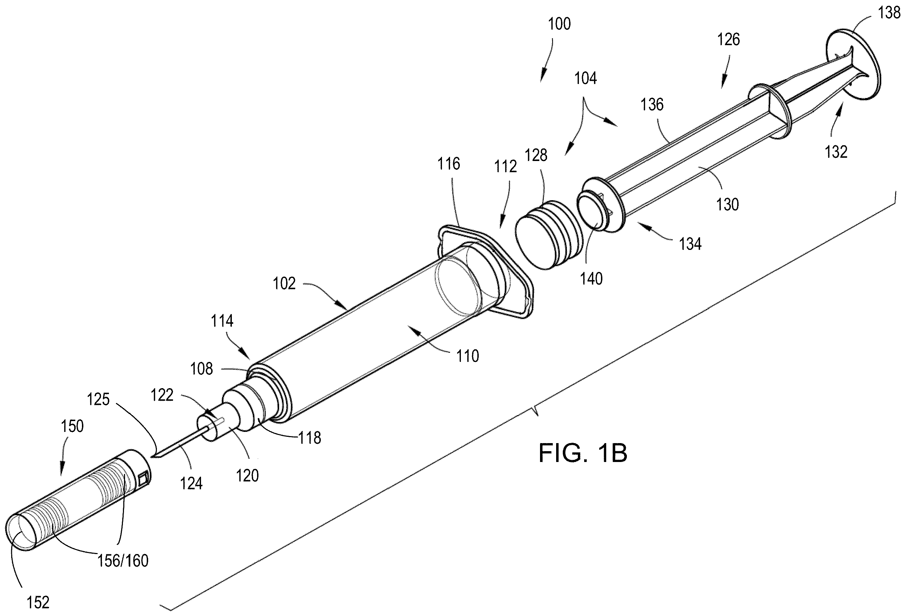

Referring to FIGS. 1A and 1B, shown is a non-limiting embodiment of a RFID-tagged medical injection device 10 with which aspects or embodiments of the disclosure may be implemented. While the medical device is shown and described hereafter as a syringe ("syringe 10"), it is recognized that other RFID-tagged medical devices, including other medical injection devices (e.g., auto-injectors) may be utilized with the system and method of the disclosure described in detail here below.

As shown in FIGS. 1A and 1B, the syringe 100 generally includes a syringe barrel 102 and a plunger assembly 104. The plunger assembly 104 is movable within the syringe barrel 102 along a longitudinal axis to an advanced position to facilitate administering of an injectable fluid (e.g., medication) to a patient, for example. The syringe barrel 102 is formed of a generally cylindrical outer wall 106 and an end member 108 that collectively define a chamber 110 for retaining fluid therein. The syringe barrel 102 includes an open proximal end 112 configured to receive the plunger assembly 104 therein and a distal end 114 at which end member 108 is positioned. The proximal end 112 of the syringe barrel 102 may include a flange 116 to facilitate handling and positioning of the syringe 100 and to maintain the relative position of the syringe barrel 102 with respect to the plunger assembly 104 during medication administration. At the distal end 114, the end member 108 may include a shoulder 118 which narrows with respect to the cylindrical outer wall 106, as well as a hub portion 120 extending out distally from shoulder 118. The hub portion 120 is formed as a partially hollow member that defines a channel 122 therethrough in fluid communication with the chamber 110. A needle 124 is attached to the hub portion 120 within channel 122, such as by being glued or otherwise secured to the hub portion 120, with the needle 124 extending distally from the hub 120 out to a distal needle tip 125.

The plunger assembly 104 of syringe 100 is formed of an elongate plunger rod 126 (more generally "plunger 126," as used hereafter) and a plunger head or stopper 128. The plunger 126 may include a main body 130 extending between a plunger proximal end 132 and a plunger distal end 134. In some embodiments, the main body 130 may include a plurality of elongate vanes or walls 136 extending axially along a length thereof between the plunger proximal end 132 and the plunger distal end 134. A thumb press 138 is positioned at the plunger proximal end 132 that may be engaged by a thumb (or other finger) of the user to apply a distally directed force to the plunger assembly 104 to move the plunger 126 with respect to the syringe barrel 102. In some embodiments, a flanged extension member 140 (e.g., disc-shaped flange) is positioned at the plunger distal end 134 that is configured to mate with the stopper 128. In other embodiments, the plunger distal end 134 may include a female receptacle formed therein that is configured to receive and connect to a protrusion (e.g., pin) extending out proximally from the stopper, with the protrusion and receptacle engaging via threading, for example.

As shown in FIGS. 1A and 1B, a needle shield 150 of syringe assembly 102 may be coupled to the syringe barrel 102 to protect the needle 124. According to aspects of the disclosure, the needle shield 150 may be composed of a rigid outer casing 152 that provides structure to the needle shield 150 and a compliant inner casing or "jupe" 154 that surrounds the needle 124 (when needle shield 150 is coupled to syringe barrel 102). In various embodiments, the outer casing 152 may be made of a rigid material such as polypropylene (PP), polyethylene (PE), polyethylene terephthalate (PET), polystyrene (PS) or polycarbonate (PC), while the inner casing 154 is made of a deformable material such as rubber (e.g., butyl rubber, isoprene rubber, latex rubber, silicone rubber) or a thermoplastic elastomer (TPE), as non-limiting examples. The outer casing 152 and inner casing 154 of needle shield 150 are formed as separate components that may be secured together to form the needle shield 150, with the casings 152, 154 formed via separate manufacturing processes - such as the outer casing 152 being formed via injection molding and the inner casing 154 being formed via injection molding (if TPE) or compression molding (if rubber). When the needle shield 150 is secured on syringe barrel 102 (i.e., on hub 120), the needle 124 extends through a cavity defined in the inner casing 154, such that the needle tip 125 protrudes through the cavity to reach the compliant material of inner casing 154 - i.e., the needle tip 125 punctures and extends into the compliant material, such that the needle tip 125 is sealed in the compliant material of the inner casing 154. With the needle tip 125 properly sealed in the compliant material of the inner casing 154, leakage of fluid out from the needle 124 may be prevented.

According to aspects and embodiments of the disclosure, the needle shield 150 may include an RFID tag 156 integrated therein that allows for identification and/or tracking of the syringe 100. That is, the RFID tag 156 may include or store information thereon regarding the contents of the syringe 100 (i.e., the medication or drug included therein, if a prefilled syringe) and the manufacturing history of the syringe and/or may provide location information to an associated reader, so as to enable tracking of the syringe 100. Accordingly, the RFID tag 156 is considered to generally include thereon a unique identifier (UID) that is specific to the syringe 100. According to aspects or embodiments of the disclosure, the RFID tag 156 may be integrated into the needle shield 150, such as by being inlaid or otherwise disposed between the outer casing 152 and the inner casing 154 of the needle shield 150. According to other embodiments, the RFID tag 156 may be applied to an outer surface of the needle shield 150. In each of these embodiments, the RFID tag 156 conforms to the surface(s) or wall(s) to or within which it is disposed, such that when the RFID tag 156 is integrated with/on a cylindrical component such as the needle shield 150, the RFID tag 156 will have a curvature that matches that of the needle shield 150.

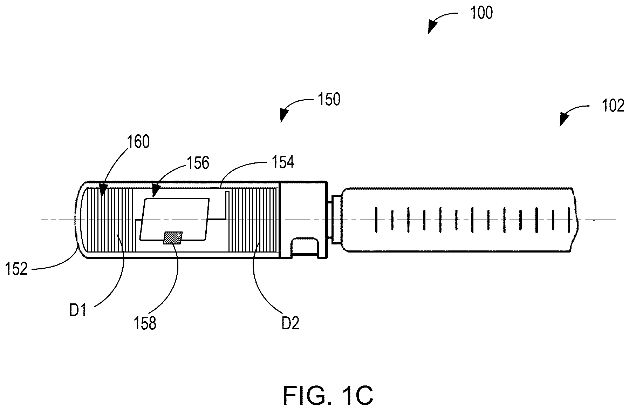

According to embodiments, and as shown in FIG. 1C, the RFID tag 156 includes an RFID chip 158 connected to an RFID dipole coupling element or antenna 160. The antenna 160 of RFID tag 156 may include two legs or dipole elements D1, D2, with the RFID chip 158 disposed between extremities of the poles D1, D2, such as being centered therebetween. In some embodiments, the RFID tag 156 may be positioned and oriented within needle shield 150 such that a dipole axis, A_{D}, of the RFID tag 156 is approximately aligned with a longitudinal axis, Ac, of the needle shield 150 (and a longitudinal axis, As, of the syringe 100), such that more strict quality controls may be implemented during manufacturing/assembly of the syringe 100 and the cap 150 thereof.

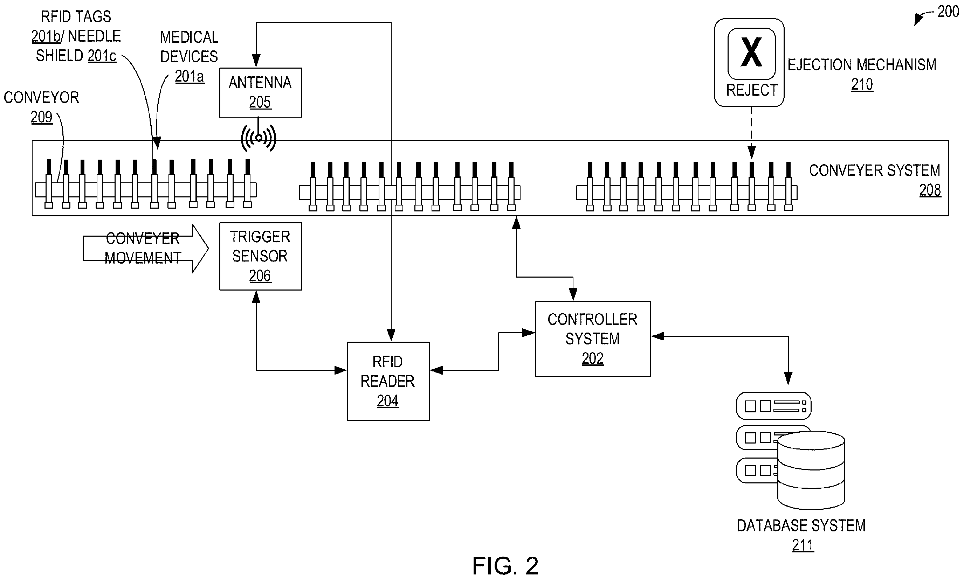

Referring now to FIG. 2, a manufacturing and reading environment or system 200 within which or by which RFID-tagged medical injection devices (such as the RFID-tagged syringes 100 of FIGS. 1A-1C) may be read is illustrated in accordance with an aspect of the disclosure. According to aspects of the disclosure, reading of the RFID-tagged medical injection devices may be implemented as part of a quality control process therefore, including detecting if a needle shield of each of the injection devices has been pierced by the needle during placement thereof onto the needle, with it recognized that such piercing of the needle shield may result in leakage that compromises the container closure integrity of the syringe.

As shown in FIG. 2, system 200 may include a plurality of medical injection devices 201a (hereafter "syringes 201a") including a plurality of RFID tags 201b integrated on a needle shield 201c thereof, a controller system 202, a RFID reader 204 including a RFID coupling element 205 (collectively a "RFID reading system"), a trigger sensor 206, a conveyor system 208 including a conveyer 209 and/or ejection mechanism 210, and a database system 211.

The plurality of RFID tags 201b on the plurality syringes 201a may include passive RFID tags, active RFID tags, or any combination thereof. The plurality of RFID tags 201b may store identifiers associated with the plurality of syringes 201a. For example, each RFID tag 201b may store a unique identifier of the syringe 201a on which that RFID tag 201b is located. As an example, an identifier may include an electronic product code (EPC).

Controller system 202 may include one or more devices capable of receiving information and/or data from RFID reader 204, trigger sensor 206, conveyor system 208, and/or database system 211 (e.g., via a communication network, via a direct wired and/or wireless connection, etc.) and/or communicating information and/or data to RFID reader 204, trigger sensor 206, conveyor system 208, and/or database system 211 (e.g., via communication network, via a direct wired and/or wireless connection, etc.). For example, controller system 202 may include a computing device, such as a server, a group of servers, and/or other like devices. In some non-limiting embodiments or aspects, controller system 202 may include middleware, such as Message Queuing Telemetry Transport (MQTT) protocol-based middleware, and/or the like, configured to manage communication with and/or control of operations of RFID reader 204, trigger sensor 206, conveyor system 208 (e.g., a programmable logic controller (PLC) of conveyor system, 208 etc.), and/or database system 211.

RFID reader 204 may include one or more devices capable of receiving information and/or data from controller system 202, trigger sensor 206, conveyor system 208, and/or database system 211 (e.g., via a communication network, via a direct wired and/or wireless connection, etc.) and/or communicating information and/or data to controller system 202, trigger sensor 206, conveyor system 208, and/or database system 211 (e.g., via communication network, via a direct wired and/or wireless connection, etc.).

RFID reader 204 may include a passive RFID reader, an active RFID reader, or any combination thereof. RFID reader 204 may be configured to read, from the plurality of RFID tags 201b on the needle shields 201c of the plurality of syringes 201a in the production line, identifiers associated with syringes 201a. For example, RFID reader 204 may be configured to attempt to read an RFID tag 201b on a syringe 201a in response to receiving a trigger signal from trigger sensor 206.

RFID coupling element 205 (which may be provided as a near field antenna or other near field coupling element, but is referred to hereafter generally as "antenna 205) of RFID reader 204 may be located proximate to conveyor 209 such that, as conveyor 209 moves the plurality of syringes 201a in the production line, the plurality of medical devices are moved one-by-one to be adjacent to (and/or to be paused adjacent to for a predetermined period of time) antenna 205 of RFID reader 204 at a "reading location", and then moved past antenna 205 of RFID reader 204. For example, a spacing between the plurality of syringes 201a in the production line on conveyor 209 and/or a size of a magnetic or electromagnetic field generated by RFID reader 204 from antenna 205 may be configured such that only a single syringe of the plurality of syringes 201a is located in the magnetic or electromagnetic field generated by RFID reader 204 from antenna 205 at any one time (e.g., such that only a single syringe of the plurality of syringes 201a can be read by RFID reader 204 at any one time - i.e. a "singulated reading"). In some embodiments, the antenna 205 may be placed from 1mm to 20mm from the RFID tag 201b when a respective syringe 201a passes the reading location.

Trigger sensor 206 may include one or more devices capable of receiving information and/or data from controller system 202, RFID reader 204, conveyor system 208, and/or database system 211 (e.g., via a communication network, via a direct wired and/or wireless connection, etc.) and/or communicating information and/or data to controller system 202, RFID reader 204, conveyor system 208, and/or database system 211 (e.g., via communication network, via a direct wired and/or wireless connection, etc.).

Trigger sensor 206 may include at least one sensor (e.g., an optical sensor, a laser sensor, etc.) configured to detect or be trigged when an individual syringe is in a predetermined location relative to antenna 205 of the RFID reader 204 (e.g., when a syringe is at the reading location, adjacent antenna 205). Trigger sensor 206 may be located proximate or adjacent to antenna 205 of RFID reader 204 and/or conveyor 209 and/or at a position relative to antenna 205 of RFID reader 204 and/or conveyor 209 at which, when a syringe is in the predetermined location relative to antenna 205 of the RFID reader 204, the syringe is within the field of detection or view of trigger sensor 206 such that trigger sensor 206 is triggered or detects the syringe. In response to being triggered or detecting a syringe in the predetermined location relative to antenna 205 of the RFID reader 204, trigger sensor 206 may be configured to communicate or transmit a trigger signal to RFID reader 204 to trigger or cause RFID reader 204 to attempt to read a RFID tag on the syringe.

Conveyor system 208 may include one or more devices capable of receiving information and/or data from controller system 202, RFID reader 204, trigger sensor 206, and/or database system 211 (e.g., via a communication network, via a direct wired and/or wireless connection, etc.) and/or communicating information and/or data to controller system 202, RFID reader 204, trigger sensor 206, and/or database system 211 (e.g., via communication network, via a direct wired and/or wireless connection, etc.).

Conveyor system 208 may include a conveyor 209 (e.g., a motorized belt conveyor, a motorized chute conveyor, a motorized roller conveyor, a motorized overhead conveyor, a motorized bucket conveyor, a motorized chain conveyor, a motorized trolley conveyor, etc.) that conveys syringes 201a along a path that is linear or circular. Each of the syringes 201a may be retained by a holding or gripping device of the conveyor 209, with the syringes 201a held in position by mechanical force, suction, or another suitable means. Conveyor 209 may be configured to individually move (e.g., move one-by-one, etc.) syringes 201a in the production line adjacent to and past antenna 205 of RFID reader 204. For example, syringes 201a may be arranged on conveyor 209, and/or conveyor 209 may be configured to move syringes 201a together in a same direction such that syringes 201a are moved one-by-one adjacent to (and/or paused adjacent to for a predetermined period of time), and moved then past, antenna 205 of RFID reader 204. As an example, conveyor 209 may include one or more high throughput production machines and/or components thereof used in the pharmaceutical industry, such as a machine used for a fill and finish process, a machine used for an automated visual inspection process, a machine used for a labelling process, a machine used for a device assembly process, and/or the like. In some embodiments, conveyor 209 may operate to provide a high-throughput for reading/testing of syringes 201a, with the conveyor 209 operating at a speed and with syringes at a pitch/spacing such that the syringes are conveyed past the antenna 205 at read rate of between 100 parts/sec to 6000 parts/min (e.g., 1000 parts/min, as a non-limiting example) - with such a read rate achievable with the conveyor 209 advancing the syringes 201a at a speed of approximately 1 m/s and at a pitch of approximately 19 mm.

Conveyor 209 may include an ejection mechanism 210 configured to remove (e.g., eject from, etc.) an individual syringe 201a from conveyor 209 and/or the production line. For example, ejection mechanism 210 may include an air blast generator, a pneumatic pusher, an overhead sweep, a pneumatic retractor, a pivot down/up and linear actuating ejector, and/or the like.

In some non-limiting embodiments or aspects, conveyor system 208 includes a programmable logic controller (PLC) configured to control one or more operations of conveyor 209. The PLC may be configured to track a sequential order of the plurality of syringes 201a in the production line and/or on conveyor 209 via one or more shift registers and/or sensors according to existing manufacturing techniques.. The PLC may be configured to use the tracked sequential order to remove or eject individual ones of the plurality of syringes 201a from the production line in response to ejection signals received from controller system 202, as described herein in more detail below.

Database system 211 may include one or more devices capable of receiving information and/or data from controller system 202, RFID reader 204, trigger sensor 206, and/or conveyor system 208 (e.g., via a communication network, via a direct wired and/or wireless connection, etc.) and/or communicating information and/or data to controller system 202, RFID reader 204, trigger sensor 206, and/or conveyor system 208 (e.g., via communication network, via a direct wired and/or wireless connection, etc.). In some non-limiting embodiments or aspects, database system 211 includes one or more local databases (e.g., local to and/or implemented by controller system 202, etc.). In other non-limiting embodiments or aspects, database system 211 includes one or more external databases (e.g., external to and/or not implemented by controller system 202, etc.). Database system 211 may be configured to store data regarding interaction between the RFID reader 204 (and antenna 205) and the RFID tags 201b of the syringes 201a - including the minimum power required by an RFID tag 201b to provide a backscattered signal, i.e., a minimum transmitted power or carrier signal strength from the RFID reader 204 (and antenna 205) required to read RFID tags 201b, as explained in further detail below.

The number and arrangement of devices shown in FIG. 2 is provided as an example. There can be additional devices, fewer devices, different devices, or differently arranged devices than those shown in FIG. 2. Furthermore, two or more devices shown in FIG. 2 can be implemented within a single device, or a single device shown in FIG. 2 can be implemented as multiple, distributed devices. Additionally, or alternatively, a set of devices (e.g., one or more devices, etc.) of system 200 can perform one or more functions described as being performed by another set of devices of system 200.

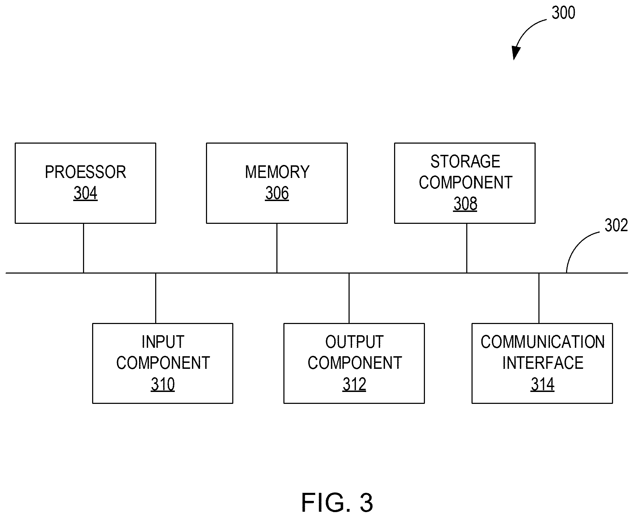

Referring now to FIG. 3, FIG. 3 is a diagram of example components of a device 300. Device 300 may correspond to one or more devices of controller system 202, RFID reader 204 (e.g., one or more devices of a system of RFID reader 204, etc.), trigger sensor 206 (e.g., one or more devices of a system of trigger sensor 206, etc.), one or more devices of conveyor system 208, and/or one or more devices of database system 211. In some non-limiting embodiments or aspects, one or more devices of controller system 202, RFID reader 204 (e.g., one or more devices of a system of RFID reader 204, etc.), trigger sensor 206 (e.g., one or more devices of a system of trigger sensor 206, etc.), one or more devices of conveyor system 208 and/or one or more devices of database system 211, may include at least one device 300 and/or at least one component of device 300. As shown in FIG. 3, device 300 may include bus 302, processor 304, memory 306, storage component 308, input component 310, output component 312, and communication interface 314.

Bus 302 may include a component that permits communication among the components of device 300. In some non-limiting embodiments or aspects, processor 304 may be specifically configured to perform one or more of the mixing aspects described in hardware, software, or a combination of hardware and software. For example, processor 304 may include a processor (e.g., a central processing unit (CPU), a graphics processing unit (GPU), an accelerated processing unit (APU), etc.), a microprocessor, a digital signal processor (DSP), and/or similar processing component (e.g., a field-programmable gate array (FPGA), an application-specific integrated circuit (ASIC), etc.) that can be programmed to perform a function. Memory 306 may include random access memory (RAM), read-only memory (ROM), and/or another type of dynamic or static storage device (e.g., flash memory, magnetic memory, optical memory, etc.) that stores information and/or instructions for use by processor 304.

Storage component 308 may store information and/or software related to the operation and use of device 300. For example, storage component 308 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 310 may include a component that permits device 300 to receive information, such as via user input (e.g., a touch screen display, a keyboard, a keypad, a mouse, a button, a switch, a microphone, etc.). Additionally or alternatively, input component 310 may include a sensor for sensing information (e.g., a global positioning system (GPS) component, an accelerometer, a gyroscope, an actuator, etc.). Output component 312 may include a component that provides output information from device 300 (e.g., a display, a speaker, one or more light-emitting diodes (LEDs), etc.).

Communication interface 314 may include a transceiver-like component (e.g., a transceiver, a separate receiver and transmitter, etc.) that enables device 300 to communicate with other devices, such as via a wired connection, a wireless connection, or a combination of wired and wireless connections. Communication interface 314 may permit device 300 to receive information from another device and/or provide information to another device. For example, communication interface 314 may include an Ethernet interface, an optical interface, a coaxial interface, an infrared interface, a radio frequency (RF) interface, a universal serial bus (USB) interface, a Wi-Fi^{®} interface, a cellular network interface, and/or the like.

Device 300 may perform one or more processes described herein. Device 300 may perform these processes based on processor 304 (e.g., a central processing unit (CPU), a graphics processing unit (GPU), etc.) executing software instructions stored by a computer-readable medium, such as memory 306 and/or storage component 308. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 306 and/or storage component 308 from another computer-readable medium or from another device via communication interface 314. When executed, software instructions stored in memory 306 and/or storage component 308 may cause processor 304 to perform one or more processes described herein. Additionally or alternatively, hardwired circuitry may be used in place of or in combination with software instructions to perform one or more processes described herein.

Memory 306 and/or storage component 308 may include data storage or one or more data structures (e.g., a database, etc.). Device 300 may be capable of receiving information from, storing information in, communicating information to, or searching information stored in the data storage or one or more data structures in memory 306 and/or storage component 308.

The number and arrangement of components shown in FIG. 3 are provided as an example. In some non-limiting embodiments or aspects, device 300 may include additional components, fewer components, different components, or differently arranged components than those shown in FIG. 3. Additionally or alternatively, a set of components (e.g., one or more components) of device 300 may perform one or more functions described as being performed by another set of components of device 300.

According to aspects of the disclosure, it is recognized that reading of the RFID tags 201b of the syringes 201a by the RFID reader 204 can be used as part of a quality control check on the syringes 201a. Specifically, reading of the RFID tags 201b of the syringes 201a can be used to detect leakage in the syringes 201a resulting from piercing of a needle shield 201c thereof, such as might occur from misalignment of the needle shield 201c relative to the needle (e.g., needle 124 in FIG. 1B) that results in the needle piercing through the needle shield 201c (i.e., through the needle shield jupe) and whereby fluid from the syringe 201a may flow out from the tip of the needle when not properly retained within the needle shield 201c (i.e., not properly inserted/contained within the needle shield jupe). The sensitivity of the RFID tag 201b in each syringe 201a can be determined in order to indirectly detect whether a syringe 201a may have a pierced needle shield condition - as it has been determined that RFID tag sensitivity is impacted by the condition of the needle shield 201c, with such impact being a function of the RFID tag being potentially damaged by misalignment/piercing of the needle.

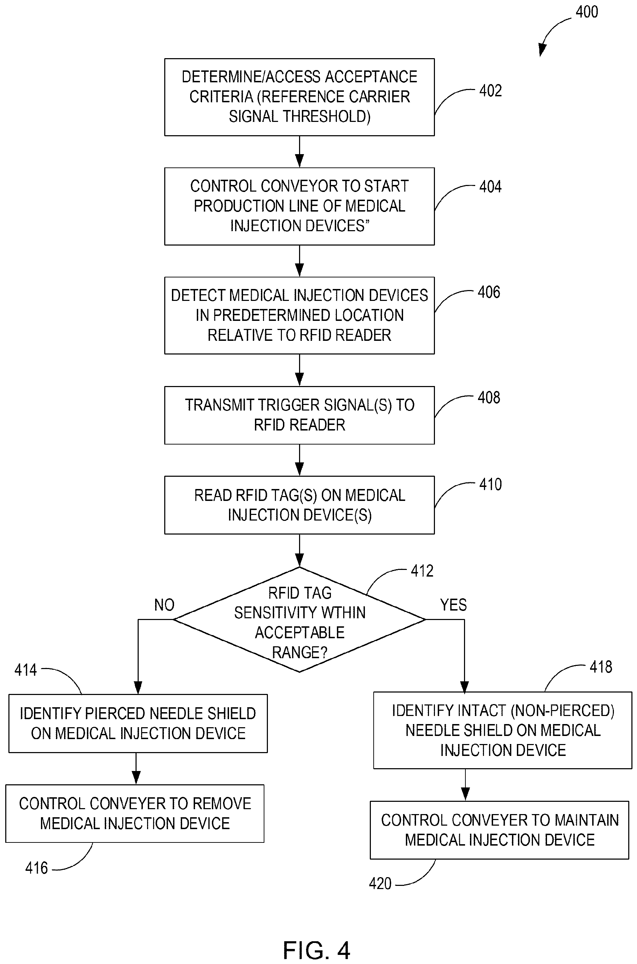

Referring now to FIG. 4, a flowchart is provided for a non-limiting embodiment or aspect of a method 400 for performing a singulated reading and testing of syringes on high-throughput conveyors (e.g., throughput of 1000 parts/min). Specifically, method 400 reads the RFID tag of each syringe as part of a quality control check that detects leakage in the syringe resulting from piercing of a needle shield thereof.

In some non-limiting embodiments or aspects, one or more of the steps of process 400 may be performed (e.g., completely, partially, etc.) by controller system 202 (e.g., one or more devices of controller system 202, etc.). In some non-limiting embodiments or aspects, one or more of the steps of process 400 may be performed (e.g., completely, partially, etc.) by another device or a group of devices separate from or including controller system 202, such as RFID reader 204 (e.g., one or more devices of a system of RFID reader 204, etc.), trigger sensor 206 (e.g., one or more devices of a system of trigger sensor 206, etc.), conveyor system 208 (e.g., one or more devices of conveyer system 208, etc.), and/or database system 211 (e.g., one or more devices of database system 211, etc.).

As shown in FIG. 4, at step 402, process 400 includes determining an acceptance criteria as to what constitutes a conforming syringe 201a versus a non-conforming syringe 201a in regards to the condition of a needle shield 201c thereof - i.e., an intact (non-pierced) needle shield 201c versus a pierced needle shield 201c. As indicated above, the sensitivity of the RFID tag 201b in each syringe 201a can be used to detect whether a syringe may have a pierced needle shield condition. Thus, according to embodiments of the disclosure, the acceptance criteria for what constitutes a conforming syringe 201a that meets product specifications can be determined by identifying the sensitivity (or a sensitivity range) of RFID tags 201b known to be included in conforming syringes 201a (i.e., a normal tag sensitivity range) - with such RFID tag sensitivity correlating to the minimum power required by an RFID tag 201b to provide a backscattered signal, i.e., a minimum transmitted power or carrier signal strength from the RFID reader 204 (and antenna 205) required to read the RFID tag 201b.

According to one aspect of the disclosure, determining of the acceptance criteria at step 402 may be performed by accessing stored data (e.g., stored in database system 211 or in memory of controller 202) regarding an established acceptance criteria. The stored data on the acceptance criteria may comprise readings previously acquired for known conforming syringes 201a (with an intact/non-pierced needle shield 201c) on the minimum transmitted power or carrier signal strength generated from the RFID reader 204 (and antenna 205) that is required to read the RFID tags 201b thereof. The data stored in database system 211 may have been acquired from previous runs of syringes 201a on conveyor system 208 and/or from separate stand-alone testing of syringes 201a when analyzing RFID tag sensitivity. According to another aspect of the disclosure, determining of the acceptance criteria at step 402 may be performed by performing an initial test run of syringes 201a on conveyor system 208 using syringes 201a known to meet product specifications (i.e., with an intact/non-pierced needle shield 201c). Data may be acquired from the test run on the minimum transmitted power or carrier signal strength from the RFID reader 204 (and antenna 205) required to read the RFID tags 201b of the syringes 201a, so as to establish the acceptance criteria, with the data then subsequently being stored in database system 211.

According to embodiments of the disclosure, for known conforming syringes 201a (with an intact/non-pierced needle shield 201c), the minimum carrier signal strength required to read the RFID tags 201b thereof (i.e., to generate a backscattered signal from the RFID tags) can be set as a reference carrier power threshold. The reference carrier power threshold can be derived for a plurality of different RFID reader settings or frequencies, such that a curve of carrier power vs. frequency can be defined for the reference carrier power threshold. As a non-limiting example of a reference carrier power threshold that may be defined for known conforming syringes 201a, the reference carrier power threshold may be -1.5dBm for a frequency of 865MHz, -5.5dBm for a frequency of 915MHz, and -1.5dBm for a frequency of 960MHz, with it being recognized that other reference carrier power thresholds may be set, as determined based on testing results from known conforming syringes 201a.

As shown in FIG. 4, upon a defining of an acceptance criteria (i.e., reference carrier power thresholds) at step 402, the process 400 continues at step 404 with controlling a conveyor to start a production line of syringes. For example, controller system 202 may control conveyor system 208 to start the production line including the plurality of syringes 201a, for example, to cause conveyor 209 to start conveyor movement to move the plurality of syringes 201a in the production line adjacent to and past antenna 205 of RFID reader 204, i.e., to the reading location. As an example, controller system 202 (e.g., middleware of controller system 202, etc.) may send a signal to conveyor system 208 (e.g., a PLC of conveyor system 208, etc.) to cause conveyor system 208 to control conveyor 209 to start conveyor movement. In such an example, controller system 202 (e.g., middleware of controller system 202, etc.) may send a signal to RFID reader to arm RFID reader 204.

As shown in FIG. 4, at step 406, process 400 includes detecting a syringe in a predetermined location relative to an antenna of a RFID reader. For example, for each syringe of the plurality of syringes 201a in the production line, trigger sensor 206 may detect when that syringe is in a predetermined location relative to antenna 205 of RFID reader 204 (i.e., at the reading location).

As shown in FIG. 4, at step 408, process 400 includes transmitting a trigger signal to a RFID reader. For example, for each syringe of the plurality of syringes 201a in the production line, trigger sensor 206 may, in response to detecting that the syringe is at a predetermined location relative to antenna 205 of RFID reader 204 in the production line (i.e., at the reading location), transmit a trigger signal to RFID reader to trigger or cause RFID reader 204 to read a RFID tag of the plurality of RFID tags 201b on that syringe 201a.

As shown in FIG. 4, at step 410, process 400 includes reading an RFID tag on a syringe. For example, for each syringe of the plurality of syringes 201a in the production line, RFID reader 204 may, in response to receiving the trigger signal from trigger sensor 206 (e.g., corresponding to that syringe, etc.), read the RFID tag 201b on that syringe 201a. As an example, RFID reader 204 may transmit radio waves via antenna 205 to RFID tags 201b, with the radio waves having a carrier power sufficient to activate the RFID tag. Responsive to receiving the radio waves, the RFID tags 201b may transmit a signal that includes an identifier associated with that syringe, back to antenna 205 to be read by RFID reader 204. As an example, RFID reader 204 may read, from the plurality of RFID tags 201b on the plurality of syringes 201a in the production line, a plurality of identifiers (e.g., a plurality of EPCs, etc.) associated with the plurality of syringes 201a (e.g. as the plurality of syringes 201a in the production line are individually moved adjacent to antenna 205 of RFID reader 204 by conveyor 209, etc.). In such an example, for each syringe of the plurality of syringes 201a in the production line, RFID reader 204 may transmit or stream, to controller system 202 (e.g., to middleware of controller system 202, etc.) the information or data read from the RFID tag (e.g., the identifier, etc.).

In addition to the RFID reader 204 transmitting information or data read from the RFID tags (e.g., the identifier, etc.) to controller system 202, the RFID reader 204 also records and transmits information or data to controller system 202 regarding the sensitivity of each of the RFID tags 201b that was read. As previously described, the RFID tag sensitivity may be measured/determined by the minimum carrier power (of radio waves/signals transmitted from the antenna 205 to each RFID tag 201b) that was needed to generate a backscattered signal response from each of the RFID tags 201b. Thus, for each syringe of the plurality of syringes 201a in the production line, RFID reader 204 may transmit or stream, to controller system 202 (e.g., to middleware of controller system 202, etc.) the minimum carrier power required to read the RFID tag 201b thereof.

As shown in FIG. 4, at step 412, process 400 includes determining whether the sensitivity of the RFID tag on each syringe that was read falls within a normal or acceptable tag sensitivity range. For example, for the RFID tag 201b of each syringe of the plurality of syringes 201a in the production line that was read by RFID reader 204, controller 202 may determine whether the sensitivity of that RFID tag 201b falls within a normal tag sensitivity range. In making such a determination, the controller 202 may compare, for each RFID tag 201b, the carrier power that was required to read that RFID tag 201b to a pre-defined reference carrier power threshold that is indicative of a minimum carrier power required to read the RFID tag in a conforming syringe 201a (i.e., with an intact/non-pierced needle shield 201c), such as was determined/retrieved at step 402. In some non-limiting embodiments or aspects, for each RFID tag 201b read by RFID reader 204, controller system 202 (e.g., middleware of controller system 202, etc.) may immediately query database system 211 that includes the stored pre-defined reference carrier power threshold, in order to compare the carrier power that was required to read each RFID tag 201b to the reference carrier power threshold.

If the carrier power that was required to read a respective RFID tag 201b is determined to be greater than the reference carrier power threshold (or otherwise outside of an acceptance criteria), the process 400 continues at step 414, by identifying the corresponding syringe 201a that includes that RFID tag 201b as having a pierced needle shield 201c. That is, if the carrier power that was required to read a respective RFID tag is determined to be greater than the reference carrier power threshold, then the sensitivity of the RFID tag is considered outside of a normal RFID tag sensitivity range, indicating that the needle of the syringe 201a has pierced through the needle shield 201c, so as to affect the sensitivity of the RFID tag 201b incorporated into the needle shield 201c.

As shown in FIG. 4, subsequent to identifying a syringe 201a as having a pierced needle shield at step 414, the process 400 continues at step 416. At step 416, in response to identifying a syringe as having a pierced needle shield, a conveyor is controlled to remove that syringe from the production line. For example, in response to identifying a syringe 201a as having a pierced needle shield, controller system 202 (e.g., middleware of controller system 202, etc.) may control (e.g., via conveyor system 208, via a PLC of conveyor system 208, etc.) conveyor 209 to remove the at least one syringe 201a from the production line. As an example, for each syringe of the plurality of syringes 201a in the production line, in response to receiving an indication that the syringe 201a has a pierced needle shield and is considered to be non-conforming, controller system 202 (e.g., middleware of controller system 202, etc.) may transmit a signal to conveyor system 208 (e.g., to a PLC of conveyor system 208, etc.) that causes conveyor system 208 to control conveyor 209 to remove that syringe from the production line (e.g., a PLC of conveyor system 208 conveyor system 208 may control ejection mechanism 210 to remove/eject that syringe from the production line).

Referring back to step 412, if the carrier power that was required to read a respective RFID tag is determined to be equal to or less than the reference carrier power threshold (or otherwise within an acceptance criteria), the process 400 continues at step 418, by identifying the corresponding syringe 201a that includes that RFID tag 201b as having an intact (non-pierced) needle shield.

As shown in FIG. 4, subsequent to identifying a syringe 201a as having an intact (non-pierced) needle shield at step 418, the process 400 continues at step 420. At step 420, in response to identifying a syringe as having an intact (non-pierced) needle shield, a conveyor is controlled to maintain that syringe on the production line. For example, in response to identifying a syringe 201a as having an intact (non-pierced) needle shield 201c, controller system 202 (e.g., middleware of controller system 202, etc.) may, in response to determining that the syringe 201a conforms with acceptance criteria and product specifications, control conveyor 209 to maintain the syringe 201a in the production line.

Beneficially, embodiments of the invention thus provide a system and method for RFID tag-based needle shield piercing and leak detection in RFID-tagged medical injection devices. The method for pierced needle shield leak detection can be performed on a high-speed throughput system (i.e., conveyor system). The sensitivity of the RFID tag in each injection device that is read by an RFID reader can be determined to indirectly detect whether the injection device may have a pierced needle shield condition, with this based on changes in the RFID tag sensitivity that result from piercing of the needle shield. The system and method can be used as a complement to legacy control systems for leak detection, such as HVLD or visual inspection systems, in order to improve the accuracy of pierced needle shield detection, or as an alternative to HVLD or visual inspection systems in environments/situations where use of such systems is not feasible.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A system for detecting needle shield piercing in each of a plurality of RFID-tagged medical injection devices having a needle shield with a RFID tag integrated therein, the system comprising:
a conveyor system configured to convey the plurality of RFID-tagged medical injection devices along a conveying path;
a RFID coupling element positioned at a reading location along the conveying path;
a RFID reader operably connected with the RFID coupling element and configured to selectively perform a singulated reading of the RFID tag of each respective RFID-tagged medical injection device upon the RFID-tagged medical injection device passing the RFID coupling element at the reading location; and
at least one processor coupled to a memory and configured to:
record, for each RFID tag read by the RFID reader, a carrier power of a signal transmitted from the RFID coupling element to the RFID tag needed to generate a backscattered signal response from the RFID tag;
compare the carrier power provided to each respective RFID tag to a specified reference carrier power threshold; and
if the carrier power provided to a respective RFID tag read by the RFID reader is greater than the reference carrier power threshold, identify the respective RFID-tagged medical injection device as having a pierced needle shield.

2. The system of claim 1, wherein the reference carrier power threshold comprises a minimum carrier power needed to generate a backscattered signal from the RFID tag, for a RFID-tagged medical injection device having an intact needle shield.

3. The system of claim 1 or claim 2, wherein the reference carrier power threshold comprises a carrier power curve of carrier power vs. frequency.

4. The system of claim 1 or claim 2, wherein the reference carrier power threshold is -1.5dBm for a frequency of 865MHz, -5.5dBm for a frequency of 915MHz, and - 1.5dBm for a frequency of 960MHz.

5. The system of any of claims 1-4, wherein the at least one processor is further configured to determine the reference carrier power threshold from a plurality of reference readings acquired from RFID-tagged medical injection devices having an intact needle shield, the reference readings being carrier powers needed to generate a backscattered signal response from the RFID tag of the RFID-tagged medical injection devices.

6. The system of any of claims 1-5, further comprising at least one trigger sensor configured to detect when an individual RFID-tagged medical injection device is at the reading location;
wherein, for each RFID-tagged medical injection device of the plurality of RFID-tagged medical injection devices on the conveyor system, the at least one sensor is configured to transmit a trigger signal to the RFID reader to cause the RFID reader to read the RFID tag of a respective RFID-tagged medical injection device in response to detecting that that RFID-tagged medical injection device is at the reading location; and
wherein the RFID reader is configured to read the RFID tag on the respective RFID-tagged medical injection device in response to receiving the trigger signal from the at least one sensor.

7. The system of any of claims 1-6, wherein in response to identifying a respective RFID-tagged medical injection device as having a pierced needle shield, the at least one processor is further configured to control the conveyor system to eject the RFID-tagged medical injection device from the conveyor system.

8. The system of any of claims 1-7, wherein the plurality of RFID-tagged medical injection devices comprise a plurality of syringes.

9. The system of any of claims 1-8, wherein the RFID coupling element comprises a near field coupling element placed from 1mm to 20mm from the RFID tag when a respective RFID-tagged medical injection device passes the reading location.

10. The system of any of claims 1-9, wherein the conveyor system is configured to convey the plurality of RFID-tagged medical devices past the reading location at a rate of up to 1000 parts/min.

11. A method for detecting needle shield piercing in each of a plurality of RFID-tagged medical injection devices having a needle shield with a RFID tag integrated therein, the method comprising:
conveying each of a plurality of RFID-tagged medical devices, via a conveyor system, along a conveying path, each of the plurality of RFID-tagged medical devices comprising an RFID tag integrated with a needle shield thereof;
providing a RFID reading system at a position along the conveying path, the RFID reading system comprising:
a RFID coupling element positioned at a reading location along the conveying path; and
a RFID reader operably connected with the RFID coupling element;
performing a singulated reading of each of the plurality of RFID-tagged medical devices via the RFID reading system, as the respective RFID-tagged medical devices pass the reading location; and
based on the reading of each of the plurality of RFID-tagged medical devices, identifying, via at least one processor, a potential needle shield piercing in each respective RFID-tagged medical injection device;
wherein, in identifying a potential needle shield piercing, the method comprises:
recording, for each RFID tag, a carrier power of a signal transmitted from the RFID coupling element to the RFID tag needed to generate a backscattered signal response from the RFID tag;
comparing the carrier power provided to each respective RFID tag to a specified reference carrier power threshold; and
if the carrier power provided to a respective RFID tag read by the RFID reader is greater than the reference carrier power threshold, identifying the respective RFID-tagged medical injection device as having a pierced needle shield.

12. The method of claim 11, wherein the reference carrier power threshold comprises a minimum carrier power needed to generate a backscattered signal from the RFID tag, for a RFID-tagged medical injection device having an intact needle shield.

13. The method of claim 11 or claim 12, further comprising controlling the conveyor system to eject a respective RFID-tagged medical injection device from the conveyor system responsive to the RFID-tagged medical injection device being identified as having a pierced needle shield.

14. The method of any of claims 11-13, further comprising:
detecting when an individual RFID-tagged medical injection device is at the reading location using at least one trigger sensor; and
in response to detecting that that RFID-tagged medical injection device is at the reading location, transmitting a trigger signal from the at least one trigger sensor to the RFID reader to cause the RFID reader to read the RFID tag of a respective RFID-tagged medical injection device.

15. The method of any of claims 11-14, further comprising positioning the RFID coupling element at the reading location so as to be spaced 1-20 mm from the RFID tag when a respective RFID-tagged medical injection device passes the reading location.

16. A system for detecting needle shield piercing in each of a plurality of RFID-tagged medical injection devices having a needle shield with a RFID tag integrated therein, the system comprising:
a conveyor system configured to convey the plurality of RFID-tagged medical injection devices along a conveying path;
a RFID coupling element positioned at a reading location along the conveying path;
a RFID reader operably connected with the RFID coupling element and configured to selectively perform a singulated reading of the RFID tag of each respective RFID-tagged medical injection device upon the RFID-tagged medical injection device passing the RFID coupling element at the reading location; and
at least one processor coupled to a memory and configured to:
determine a tag sensitivity of the RFID tag in each of the RFID-tagged medical injection devices read by the RFID reader;
compare the tag sensitivity of each RFID tag to a pre-determined normal tag sensitivity range; and
if the tag sensitivity for a respective RFID tag is outside of the normal tag sensitivity range, identify the respective RFID-tagged medical injection device as having a pierced needle shield.

17. The system of claim 16, wherein the at least one processor is further configured to determine the normal tag sensitivity range from a plurality of reference readings acquired from RFID-tagged medical injection devices having an intact needle shield.

18. The system of claim 16 or claim 17, wherein in determining the tag sensitivity, the at least one processor is configured to record, for each RFID tag read by the RFID reader, a carrier power of a signal transmitted from the RFID coupling element to the RFID tag needed to generate a backscattered signal response from the RFID tag, and wherein in comparing the tag sensitivity of each RFID tag to the pre-determined normal tag sensitivity range, the at least one processor is configured to compare the carrier power to a specified reference carrier power threshold, with the respective RFID-tagged medical injection device being identified as having a pierced needle shield when the recorded carrier power for the respective RFID tag read by the RFID reader is greater than the reference carrier power threshold.

19. The system of any of claims 16-18, wherein in response to identifying a respective RFID-tagged medical injection device as having a pierced needle shield, the at least one processor is further configured to control the conveyor system to eject the RFID-tagged medical injection device from the conveyor system.

20. The system of any of claims 16-19, wherein the RFID coupling element comprises a near field coupling element placed from 1mm to 20mm from the RFID tag when a respective RFID-tagged medical injection device passes the reading location.
